# EUROPEAN PATENT APPLICATION

(11) **EP 4 674 404 A1**
(43) Date of publication of application: **07.01.2026**
(21) Application number: 24763986.7
(22) Date of filing: 28.02.2024
(51) Int. Cl.: A61K 8/34, A61K 8/06, A61K 8/27, A61K 8/29, A61K 8/36, A61K 8/37, A61K 8/42, A61Q 17/04

(54) **EMULSION COMPOSITION**

(30) Priority: 28.02.2023 JP 2023030390
(71) Applicant: AJINOMOTO CO., INC., Chuo-ku Tokyo 104-8315 (JP)
(72) Inventor: YAMAZAKI, Tsuyoshi, Kawasaki-shi, Kanagawa 210-8681 (JP)
(74) Representative: Mewburn Ellis LLP
(86) International application number: PCT/JP2024/007352
(87) International publication number: WO 2024/181508

(57) **Abstract**

The present invention relates to a liquid emulsion composition containing (A) an oil gelling agent having an amide group, (B) one or more selected from the group consisting of an ultraviolet absorber and an ultraviolet scattering agent, (C) one or more selected from the group consisting of divalent alcohol having 4 to 6 carbon atoms, fatty acid that is liquid at ordinary temperature, aliphatic alcohol that is liquid at ordinary temperature, an ester of straight chain saturated fatty acid and monovalent alcohol that is liquid at ordinary temperature, and an ester of aromatic carboxylic acid that is liquid at ordinary temperature, (D) an emulsifier, and (E) water.

The present invention can provide an emulsion composition that has improved UV protective effect and is superior in stability and feel of use. In addition, the emulsion composition of the present invention can also be provided as a sunscreen preparation that does not use silicone-based materials or petroleum-based polymers.

## Description

### [Technical Field]

The present invention relates to a liquid emulsion composition having improved ultraviolet protective effect.

### [Background Art]

Emulsion compositions consist of water and oil and form the backbone of formulations of many cosmetic preparations, and also form the backbone of formulations of sunscreen preparations. In particular, sunscreen preparations require a UV protective effect as a function unique to the preparations, in addition to texture and emulsification stability. UV protective effect refers to protection against long-wavelength ultraviolet rays (UVA) and medium wavelength ultraviolet rays (UVB), which are classified by wavelength. This protective effect is achieved by incorporating an ultraviolet absorber and an ultraviolet scattering agent into the preparation.

However, even if attempts are made to enhance the UV protective effect by incorporating large amounts of ultraviolet absorbers, national regulations often impose upper limits on the amount of ultraviolet absorber to be used, which often makes it difficult to incorporate an amount sufficient to improve UV protective effect. Furthermore, incorporating large amounts of ultraviolet scattering agents, such as titanium oxide and zinc oxide, can lead to a problem of noticeable emergence of creaking feel and a white residue after application. Therefore, to maximize the effectiveness of ultraviolet absorbers and ultraviolet scattering agents blended in sunscreen preparations, combinations with raw materials that may improve UV protective effect are being considered.

For example, it has been disclosed that the sun protection factor (SPF) value of a sunscreen composition can be improved by combining a poly(oxyethylene-oxypropylene)methylpolysiloxane copolymer wax with a specific organic ultraviolet absorber (Patent Literature 1).

Furthermore, it has been disclosed that the UV protective effect, particularly SPF value, was improved by preparing an oil-in-water type emulsion cosmetic containing a carboxylic acid-modified silicone that is liquid at 50°C, a vinyl polymer emulsion, a basic compound, and an inorganic UV protective agent consisting of hydrophobic fine particle metal oxide (Patent Literature 2).

Furthermore, it has been proposed to use lipoamino acid alkyl esters (fatty acid acylamino acid alkyl esters) as SPF improvers (Patent Literature 3).

However, the technique disclosed in Patent Literature 1 was difficult to adapt in the recent tendency toward an increase in the number of consumers seeking silicone-free products.

The technique disclosed in Patent Literature 2 was limited to oil-in-water type emulsion cosmetics with insufficient water resistance, and had a problem of a tendency to avoid use of petroleum-based polymers, given a market demand for plastic-free products.

Furthermore, the technique disclosed in Patent Literature 3 had a problem of difficulty in ensuring formulation stability and maintaining good texture, because lipoamino acid alkyl ester, which is an oily raw material, was preferably blended in 0.5 wt% to 20 wt%.

Therefore, there was a demand for a technique that could improve the UV protective effect of sunscreen preparations without using silicone-based materials or petroleum-based polymers, and without impairing stability or feel of use.

### [Citation List]

### [Patent Literature]

[Patent Literature 1]
   JP 2009-508858 A
[Patent Literature 2]
   WO 2020/036064
[Patent Literature 3]
   JP 2022-091735 A

### [Summary of Invention]

### [Technical Problem]

Therefore, an object of the present invention is to provide an emulsion composition that has improved UV protective effect, is superior in stability and feel of use, and can also be used as a sunscreen preparation that does not use silicone-based materials or petroleum-based polymers.

### [Solution to Problem]

The present inventor has conducted intensive studies in an attempt to solve the above-mentioned problems and found that a liquid emulsion composition containing (A) an oil gelling agent having an amide group, (B) one or more selected from the group consisting of an ultraviolet absorber and an ultraviolet scattering agent, (C) one or more selected from the group consisting of divalent alcohol having 4 to 6 carbon atoms, fatty acid that is liquid at ordinary temperature, aliphatic alcohol that is liquid at ordinary temperature, an ester of straight chain saturated fatty acid and monovalent alcohol that is liquid at ordinary temperature, and an ester of aromatic carboxylic acid that is liquid at ordinary temperature, (D) an emulsifier, and (E) water affords an emulsion composition that has improved UV protective effect, good emulsion stability, and good feel of use. The present inventor has conducted further studies and completed the present invention.

That is, the present invention relates to the following.
[1] A liquid emulsion composition comprising (A) an oil gelling agent having an amide group, (B) one or more selected from the group consisting of an ultraviolet absorber and an ultraviolet scattering agent, (C) one or more selected from the group consisting of divalent alcohol having 4 to 6 carbon atoms, fatty acid that is liquid at ordinary temperature, aliphatic alcohol that is liquid at ordinary temperature, an ester of straight chain saturated fatty acid and monovalent alcohol that is liquid at ordinary temperature, and an ester of aromatic carboxylic acid that is liquid at ordinary temperature, (D) an emulsifier, and (E) water.
[2] The emulsion composition of [1], wherein the (A) oil gelling agent having an amide group is an N-acylamino acid dialkylamide represented by the following formula (I): 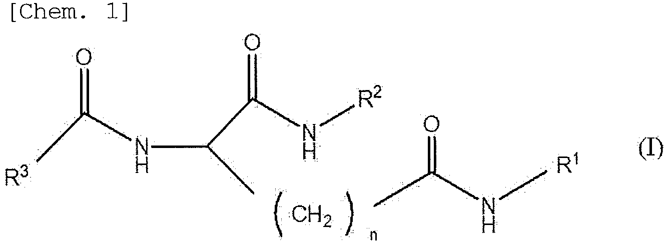 in the formula (I), R¹ and R² are the same or different and each is an alkyl group having 1 to 12 carbon atoms, R³ is a hydrocarbon group having 5 to 21 carbon atoms, and n is 1 or 2.
[3] The emulsion composition of [2], wherein the (A) oil gelling agent having an amide group is one or more selected from the group consisting of N-lauroyl-L-glutamic acid dibutylamide and N-2-ethylhexanoyl-L-glutamic acid dibutylamide.
[4] The emulsion composition of any of [1] to [3], wherein a content of the (A) oil gelling agent having an amide group is 0.01 wt% to 3 wt%.
[5] The emulsion composition of any of [1] to [4], wherein the ultraviolet absorber is an oil-soluble ultraviolet absorber.
[6] The emulsion composition of any of [1] to [5], wherein the ultraviolet scattering agent is one or more selected from the group consisting of zinc oxide, titanium oxide, fine particle zinc oxide, fine particle titanium oxide, silica-treated zinc oxide, silica-treated titanium oxide, silica-treated fine particle zinc oxide, silica-treated fine particle titanium oxide, silicone-treated zinc oxide, silicone-treated titanium oxide, silicone-treated fine particle zinc oxide, silicone-treated fine particle titanium oxide, N-stearoyl-L-glutamic acid-treated zinc oxide, N-stearoyl-L-glutamic acid-treated titanium oxide, N-stearoyl-L-glutamic acid-treated fine particle zinc oxide, N-stearoyl-L-glutamic acid-treated fine particle titanium oxide, N-lauroyl-L-lysine-treated zinc oxide, N-lauroyl-L-lysine-treated titanium oxide, N-lauroyl-L-lysine-treated fine particle zinc oxide, and N-lauroyl-L-lysine-treated fine particle titanium oxide.
[7] The emulsion composition of any of [1] to [6], wherein the (C) one or more selected from the group consisting of divalent alcohol having 4 to 6 carbon atoms, fatty acid that is liquid at ordinary temperature, aliphatic alcohol that is liquid at ordinary temperature, an ester of straight chain saturated fatty acid and monovalent alcohol that is liquid at ordinary temperature, and an ester of aromatic carboxylic acid that is liquid at ordinary temperature is one or more selected from the group consisting of 1,3-butanediol, 1,2-pentanediol, 1,2-hexanediol, 14-methylpentadecanoic acid, 16-methylheptadecanoic acid, 9-hexadecenoic acid, cis-9-octadecenoic acid, 2-hexyl-1-decanol, 16-methylheptadecanol, 2-octyl-1-dodecanol, 2-decyl-1-tetradecanol, cis-9-octadecen-1-ol, isopropyl myristate, isopropyl palmitate, and alkyl(C12-15) benzoate.
[8] The emulsion composition of any of [1] to [7], wherein a content ratio of the component (A) and component (D) [(A):(D)] is 1:30 to 30:1 in weight ratio.
[9] The emulsion composition of any of [1] to [8], further comprising (F) oily component.
[10] The emulsion composition of any of [1] to [9], which is an oil-in-water type emulsion composition.
[11] The emulsion composition of any of [1] to [9], which is a water-in-oil type emulsion composition.
[12] A method for improving a UV protective effect of an emulsion composition, comprising preparing an emulsion composition (1) comprising (B) one or more selected from the group consisting of an ultraviolet absorber and an ultraviolet scattering agent, (D) an emulsifier, and (E) water, dissolving (A) an oil gelling agent having an amide group in (C) one or more selected from the group consisting of divalent alcohol having 4 to 6 carbon atoms, fatty acid that is liquid at ordinary temperature, aliphatic alcohol that is liquid at ordinary temperature, an ester of straight chain saturated fatty acid and monovalent alcohol that is liquid at ordinary temperature, and an ester of aromatic carboxylic acid that is liquid at ordinary temperature, adding the solution to the aforementioned emulsion composition (1), and mixing them to obtain a liquid emulsion composition (2).
[13] The improvement method of [12], wherein the (A) oil gelling agent having an amide group is an N-acylamino acid dialkylamide represented by the following formula (I): 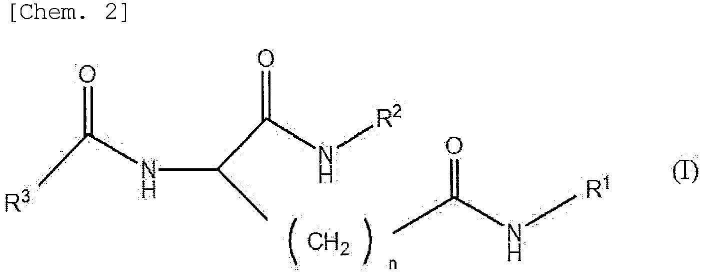 in the formula (I), R¹ and R² are the same or different and each is an alkyl group having 1 to 12 carbon atoms, R³ is a hydrocarbon group having 5 to 21 carbon atoms, and n is 1 or 2.
[14] The improvement method of [13], wherein the (A) oil gelling agent having an amide group is one or more selected from the group consisting of N-lauroyl-L-glutamic acid dibutylamide and N-2-ethylhexanoyl-L-glutamic acid dibutylamide.
[15] The improvement method of any of [12] to [14], wherein the (A) oil gelling agent having an amide group is added such that a content thereof with respect to the total amount of the liquid emulsion composition (2) is 0.01 wt% to 3 wt%.
[16] The improvement method of any of [12] to [15], wherein the ultraviolet absorber is an oil-soluble ultraviolet absorber.
[17] The improvement method of any of [12] to [16], wherein the ultraviolet scattering agent is one or more selected from the group consisting of zinc oxide, titanium oxide, fine particle zinc oxide, fine particle titanium oxide, silica-treated zinc oxide, silica-treated titanium oxide, silica-treated fine particle zinc oxide, silica-treated fine particle titanium oxide, silicone-treated zinc oxide, silicone-treated titanium oxide, silicone-treated fine particle zinc oxide, silicone-treated fine particle titanium oxide, N-stearoyl-L-glutamic acid-treated zinc oxide, N-stearoyl-L-glutamic acid-treated titanium oxide, N-stearoyl-L-glutamic acid-treated fine particle zinc oxide, N-stearoyl-L-glutamic acid-treated fine particle titanium oxide, N-lauroyl-L-lysine-treated zinc oxide, N-lauroyl-L-lysine-treated titanium oxide, N-lauroyl-L-lysine-treated fine particle zinc oxide, and N-lauroyl-L-lysine-treated fine particle titanium oxide.
[18] The improvement method of any of [12] to [17], wherein the (C) one or more selected from the group consisting of divalent alcohol having 4 to 6 carbon atoms, fatty acid that is liquid at ordinary temperature, aliphatic alcohol that is liquid at ordinary temperature, an ester of straight chain saturated fatty acid and monovalent alcohol that is liquid at ordinary temperature, and an ester of aromatic carboxylic acid that is liquid at ordinary temperature is one or more selected from the group consisting of 1,3-butanediol, 1,2-pentanediol, 1,2-hexanediol, 14-methylpentadecanoic acid, 16-methylheptadecanoic acid, 9-hexadecenoic acid, cis-9-octadecenoic acid, 2-hexyl-1-decanol, 16-methylheptadecanol, 2-octyl-1-dodecanol, 2-decyl-1-tetradecanol, cis-9-octadecen-1-ol, isopropyl myristate, isopropyl palmitate, and alkyl(C12-15) benzoate.
[19] The improvement method of any of [12] to [18], wherein a content ratio of component (A) and component (C) [(A):(C)] with respect to the total amount of the liquid emulsion composition (2) is 3:5 - 1:20 in weight ratio.
[20] The improvement method of any of [12] to [19], wherein the liquid emulsion composition (2) further comprises (F) oily component.
[21] The improvement method of any of [12] to [20], wherein the liquid emulsion composition (2) is an oil-in-water type emulsion composition.
[22] The improvement method of any of [12] to [20], wherein the liquid emulsion composition (2) is a water-in-oil type emulsion composition.
[23] Use of (A) an oil gelling agent having an amide group for improving a UV protective effect in a liquid emulsion composition comprising (B) one or more selected from the group consisting of an ultraviolet absorber and an ultraviolet scattering agent, (C) one or more selected from the group consisting of divalent alcohol having 4 to 6 carbon atoms, fatty acid that is liquid at ordinary temperature, aliphatic alcohol that is liquid at ordinary temperature, an ester of straight chain saturated fatty acid and monovalent alcohol that is liquid at ordinary temperature, and an ester of aromatic carboxylic acid that is liquid at ordinary temperature, (D) an emulsifier, and (E) water.
[24] The use of [23], wherein the (A) oil gelling agent having an amide group is dissolved in (C) one or more selected from the group consisting of divalent alcohol having 4 to 6 carbon atoms, fatty acid that is liquid at ordinary temperature, aliphatic alcohol that is liquid at ordinary temperature, an ester of straight chain saturated fatty acid and monovalent alcohol that is liquid at ordinary temperature, and an ester of aromatic carboxylic acid that is liquid at ordinary temperature, and then added.
[25] The use of [23] or [24], wherein the (A) oil gelling agent having an amide group is an N-acylamino acid dialkylamide represented by the following formula (I): 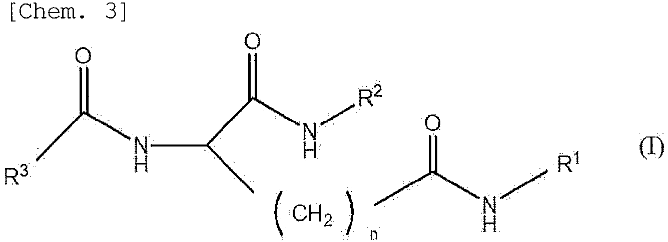 in the formula (I), R¹ and R² are the same or different and each is an alkyl group having 1 to 12 carbon atoms, R³ is a hydrocarbon group having 5 to 21 carbon atoms, and n is 1 or 2.
[26] The use of [25], wherein the (A) oil gelling agent having an amide group is one or more selected from the group consisting of N-lauroyl-L-glutamic acid dibutylamide and N-2-ethylhexanoyl-L-glutamic acid dibutylamide.
[27] The use of any of [23] to [26], wherein the (A) oil gelling agent having an amide group is added such that a content thereof with respect to the total amount of the liquid emulsion composition is 0.01 wt% to 3 wt%.
[28] The use of any of [23] to [27], wherein the ultraviolet absorber is an oil-soluble ultraviolet absorber.
[29] The use of any of [23] to [28], wherein the ultraviolet scattering agent is one or more selected from the group consisting of zinc oxide, titanium oxide, fine particle zinc oxide, fine particle titanium oxide, silica-treated zinc oxide, silica-treated titanium oxide, silica-treated fine particle zinc oxide, silica-treated fine particle titanium oxide, silicone-treated zinc oxide, silicone-treated titanium oxide, silicone-treated fine particle zinc oxide, silicone-treated fine particle titanium oxide, N-stearoyl-L-glutamic acid-treated zinc oxide, N-stearoyl-L-glutamic acid-treated titanium oxide, N-stearoyl-L-glutamic acid-treated fine particle zinc oxide, N-stearoyl-L-glutamic acid-treated fine particle titanium oxide, N-lauroyl-L-lysine-treated zinc oxide, N-lauroyl-L-lysine-treated titanium oxide, N-lauroyl-L-lysine-treated fine particle zinc oxide, and N-lauroyl-L-lysine-treated fine particle titanium oxide.
[30] The use of any of [23] to [29], wherein the (C) one or more selected from the group consisting of divalent alcohol having 4 to 6 carbon atoms, fatty acid that is liquid at ordinary temperature, aliphatic alcohol that is liquid at ordinary temperature, an ester of straight chain saturated fatty acid and monovalent alcohol that is liquid at ordinary temperature, and an ester of aromatic carboxylic acid that is liquid at ordinary temperature is one or more selected from the group consisting of 1,3-butanediol, 1,2-pentanediol, 1,2-hexanediol, 14-methylpentadecanoic acid, 16-methylheptadecanoic acid, 9-hexadecenoic acid, cis-9-octadecenoic acid, 2-hexyl-1-decanol, 16-methylheptadecanol, 2-octyl-1-dodecanol, 2-decyl-1-tetradecanol, cis-9-octadecen-1-ol, isopropyl myristate, isopropyl palmitate, and alkyl(C12-15) benzoate.
[31] The use of any of [23] to [30], wherein a content ratio of component (A) and component (C) [(A):(C)] with respect to the total amount of the liquid emulsion composition is 3:5 - 1:20 in weight ratio.
[32] The use of any of [23] to [31], wherein the liquid emulsion composition further comprises (F) oily component.
[33] The use of any of [23] to [32], wherein the liquid emulsion composition is an oil-in-water type emulsion composition.
[34] The use of any of [23] to [32], wherein the liquid emulsion composition is a water-in-oil type emulsion composition.

### [Advantageous Effects of Invention]

The present invention can provide an emulsion composition that has improved UV protective effect and is superior in emulsification stability and feel of use.

The emulsion composition of the present invention can also be provided as a sunscreen preparation that does not use silicone-based materials or petroleum-based polymers.

### [Description of Embodiments]

The present invention provides a liquid emulsion composition with improved UV protective effect (hereinafter also to be referred to as "the emulsion composition of the present invention" in the present specification).

The emulsion composition of the present invention contains (A) an oil gelling agent having an amide group, (B) one or more selected from the group consisting of an ultraviolet absorber and an ultraviolet scattering agent, (C) one or more selected from the group consisting of divalent alcohol having 4 to 6 carbon atoms, fatty acid that is liquid at ordinary temperature, aliphatic alcohol that is liquid at ordinary temperature, an ester of straight chain saturated fatty acid and monovalent alcohol that is liquid at ordinary temperature, and an ester of aromatic carboxylic acid that is liquid at ordinary temperature, (D) an emulsifier, and (E) water.

An oil gelling agent having an amide group which is contained as component (A) in the present invention is not particularly limited as long as it has one or more amido groups in the molecular structure and can gelatinize oily components. An amino acid-based oil gelling agent is preferably used, and a preferred example of the amino acid-based oil gelling agent is N-acylamino acid alkylamide.

The N-acylamino acid alkylamide used in the present invention can be produced, for example, by reacting a long-chain fatty acid halide with an amino acid by the Schotten-Baumann reaction in the presence of a basic catalyst to form an N-acylamino acid, and then reacting same by heating with an amine derivative such as alkylamine in the presence or absence of an acid catalyst. Alternatively, it can be produced by reacting an amino acid with an amine derivative such as alkylamine in the presence or absence of an acid catalyst, and then N-acylating the obtained amino acid amide with an acylating agent such as fatty acid halide.

As for the N-acylalkylamide derivative of the amino acid having an optical isomer, any of the L-form, D-form, and DL-form can be used, and an N-acylalkylamide derivative in L-form can be more preferably used.

For the object of the present invention, an N-acylamino acid dialkylamide represented by the following formula (I) is more preferably used as the N-acylamino acid alkylamide: 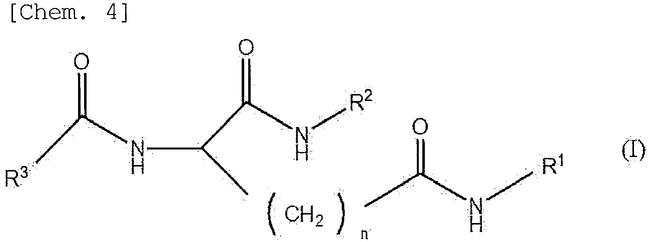 in the formula (I), R¹ and R² are the same or different and each is an alkyl group having 1 to 12 carbon atoms, R³ is a hydrocarbon group having 5 to 21 carbon atoms, and n is 1 or 2.

In the formula (I), the alkyl groups having 1 to 12 carbon atoms represented by R¹ and R² may be the same or different, and may be a straight chain alkyl group or a branched alkyl group. Specific examples include methyl, ethyl, n-propyl, 1-methylethyl (isopropyl), n-butyl, 1-methylpropyl, 2-methylpropyl (isobutyl), 1,1-dimethylethyl (tert-butyl), n-pentyl, 3-methylbutyl (isopentyl), 1,1-dimethylpropyl, n-hexyl, 4-methylpentyl (isohexyl), n-heptyl, n-octyl, 6-methylheptyl (isooctyl), 2-ethylhexyl, n-nonyl, n-decyl, n-undecyl, and n-dodecyl.

From the aspect of improving UV protective effect, the above-mentioned group represented by R¹ or R² is preferably straight chain or branched alkyl having 2 to 6 carbon atoms, more preferably straight chain or branched alkyl having 3 to 5 carbon atoms, most preferably n-butyl.

In the formula (I), the hydrocarbon group having 5 to 21 carbon atoms represented by R³ may be saturated or unsaturated, and may be any of a straight chain hydrocarbon group, a branched chain hydrocarbon group, and a cyclic hydrocarbon group. Therefore, examples of the acyl group represented by R³-CO- in the formula (I) include n-hexanoyl, n-octanoyl, 2-ethylhexanoyl, n-nonanoyl, n-decanoyl, 10-undecenoyl, n-dodecanoyl (lauroyl), n-tetradecanoyl (myristoyl), n-hexadecanoyl (palmitoyl), 14-methylpentadecanoyl (isopalmitoyl), n-octadecanoyl (stearoyl), 16-methylheptadecanoyl (isostearoyl), cis-9-octadecenoyl (oleoyl), docosanoyl (behenoyl), benzoyl, and the like.

From the aspect of improving UV protective effect, the above-mentioned acyl group is preferably a straight chain or branched alkanoyl group having 8 to 18 carbon atoms or a straight chain or branched alkenoyl group having 8 to 18 carbon atoms, more preferably a straight chain or branched alkanoyl group having 8 to 12 carbon atoms, particularly preferably n-dodecanoyl or 2-ethylhexanoyl.

In the formula (I), when n is 1, the acidic amino acid residue in the N-acylamino acid dialkylamide represented by the formula (I) is L-aspartic acid, and when n is 2, the aforementioned acidic amino acid residue is L-glutamic acid.

From the aspect of showing high gelling ability for oily substances, n is preferably 2.

Specific examples of the N-acylamino acid dialkylamide represented by the above-mentioned formula (I) include N-octanoyl-L-glutamic acid dibutylamide, N-2-ethylhexanoyl-L-glutamic acid dibutylamide, N-decanoyl-L-glutamic acid dibutylamide, and N-lauroyl-L-glutamic acid dibutylamide, and N-2-ethylhexanoyl-L-glutamic acid dibutylamide and N-lauroyl-L-glutamic acid dibutylamide are particularly preferred examples.

The N-acylamino acid dialkylamide represented by the above-mentioned formula (I) may further have one or more asymmetric carbons depending on the kind of R¹, R², and R³. Any stereoisomers such as optical isomers and diastereomers based on such asymmetric carbons may be used, and a racemate or a mixture of any stereoisomers can also be used.

Furthermore, when one or more of R¹, R², and R³ have an olefinic double bond, either of the Z- or E-form geometric isomers resulting from the configuration thereof may be used, or a mixture of any geometric isomers may also be used.

Furthermore, the N-acylamino acid dialkylamide represented by the above-mentioned formula (I) may be a hydrate or a crystal in any form.

In the emulsion composition of the present invention, as component (A), one kind of the above-mentioned N-acylamino acid dialkylamide may be selected and used alone, or two or more kinds thereof may be selected and used in combination.

From the aspect of improving UV protective effect, it is preferable to select two or more kinds of N-acylamino acid dialkylamide represented by the above-mentioned formula (I) and use them in combination as component (A), and it is particularly preferable to use N-2-ethylhexanoyl-L-glutamic acid dibutylamide and N-lauroyl-L-glutamic acid dibutylamide in combination.

The N-acylamino acid dialkylamide represented by the above-mentioned formula (I) may be produced by a production method known per se, for example, a method that combines the above-mentioned N-acylation reaction of amino acids by the Schotten-Baumann reaction and the amidation reaction with alkylamines or the like, and used. In addition, a commercially available product provided by Ajinomoto Co., Inc. and the like can also be used.

In the emulsion composition of the present invention, the content of (A) oil gelling agent having an amide group is preferably 0.01 wt% to 3 wt%. When the content of component (A) is less than 0.01 wt%, the UV protective effect may not be improved. On the other hand, when the content of component (A) exceeds 3 wt%, a liquid emulsion composition may not be obtained.

From the aspect of stability of an emulsion composition and improving UV protective effect, the content of component (A) is more preferably 0.05 wt% or more, further preferably 0.1 wt% or more, still more preferably 0.4 wt% or more, further more preferably 0.5 wt% or more.

From the aspect of stably obtaining a liquid emulsion composition, the content of component (A) is more preferably 2.9 wt% or less, further preferably 1.9 wt% or less.

As the ultraviolet absorber contained as component (B) in the emulsion composition of the present invention, any ultraviolet absorber can be used without particular limitation as long as it has absorption ability in the ultraviolet region and can be used in skin compositions such as pharmaceutical product, cosmetics and the like.

Examples of absorber for medium wavelength ultraviolet rays (UVB) include cinnamic acid derivatives such as 2-ethylhexyl paramethoxycinnamate and 2-ethoxyethyl 3-(4-methoxyphenyl)propenoate (cinoxate); salicylic acid derivatives such as 2-ethylhexyl salicylate, homomenthyl salicylate, and 3,3,5-trimethylcyclohexyl salicylate (homosalate); triazine derivatives such as 2,4,6-tris[4-(2-ethylhexyloxycarbonyl)anilino]-1,3,5-triazine; 2-phenyl-1H-benzimidazole-5-sulfonic acid; benzylidene camphor derivatives such as 3-(4-methylbenzylidene)-1,7,7-trimethylbicyclo[2.2.1]heptan-2-one (methylbenzylidene camphor); hydantoin derivatives such as 2-ethylhexyl dimethoxybenzylidene dioxoimidazolidine propionate; 1-(3,4-dimethoxyphenyl)-4,4-dimethyl-1,3-pentanedione; silicone-based UV absorbers such as diethyl 4-propynyloxybenzalmalonate-modified dimethylpolysiloxane (Polysilicone-15); and the like.

Examples of absorber for long-wavelength ultraviolet rays (UVA) include dibenzoylmethane derivatives such as 4-tert-butyl-4'-methoxydibenzoylmethane; benzoic acid derivatives such as 2-[4-(diethylamino)-2-hydroxybenzoyl]benzoic acid hexyl ester; benzylidene camphor derivatives such as terephthalylidene dicamphor sulfonic acid; and the like.

Examples of absorber for medium wavelength ultraviolet rays (UVB) and long-wavelength ultraviolet rays (UVA) include benzophenone derivatives such as 2-hydroxy-4-methoxybenzophenone (oxybenzone) and 2-hydroxy-4-methoxybenzophenone sulfonic acid; 2-cyano-3,3-diphenyl-2-propenoic acid 2-ethylhexyl ester (octocrylene); hydroxyphenylbenzotriazole derivatives such as 2-(2H-indazol-2-yl)-4-methyl-6-[2-methyl-3-[methyldi(trimethylsilyloxy)silyl]propyl]phenol; phenyltriazine derivatives such as 2,4-bis-[{4-(2-ethylhexyloxy)-2-hydroxy}phenyl]-6-(4-methoxyphenyl)-1,3,5-triazine; cinnamic acid derivatives such as 3-(3-methoxy-4-hydroxyphenyl)propenoic acid (ferulic acid); and methylene bis-benzotriazolyl tetramethylbutylphenol; and the like.

In the present invention, oil-soluble ultraviolet absorbers are preferably used, and oil-soluble cinnamic acid derivatives, salicylic acid derivatives, triazine derivatives, benzylidene camphor derivatives, hydantoin derivatives, dibenzoylmethane derivatives, benzoic acid derivatives and phenyltriazine derivatives, as well as 2-cyano-3,3-diphenyl-2-propenoic acid 2-ethylhexyl ester (octocrylene), 1-(3,4-dimethoxyphenyl)-4,4-dimethyl-1,3-pentanedione, methylene bis-benzotriazolyl tetramethylbutylphenol and the like are more preferably used. In addition, 2-ethylhexyl paramethoxycinnamate, 2-ethoxyethyl 3-(4-methoxyphenyl)propenoate (cinoxate), 2-ethylhexyl salicylate, 4-tert-butyl-4'-methoxydibenzoylmethane, 2-[4-(diethylamino)-2-hydroxybenzoyl]benzoic acid hexyl ester, 2-cyano-3,3-diphenyl-2-propenoic acid 2-ethylhexyl ester (octocrylene), 2,4,6-tris[4-(2-ethylhexyloxycarbonyl)anilino]-1,3,5-triazine, 2,4-bis-[{4-(2-ethylhexyloxy)-2-hydroxy}-phenyl]-6-(4-methoxyphenyl)-1,3,5-triazine, 2-ethylhexyl dimethoxybenzylidene dioxoimidazolidine propionate, 1-(3,4-dimethoxyphenyl)-4,4-dimethyl-1,3-pentanedione, 3-(4-methylbenzylidene)-1,7,7-trimethylbicyclo[2.2.1]heptan-2-one (methylbenzylidene camphor), methylene bis-benzotriazolyl tetramethylbutylphenol, and the like are further preferably used.

As the ultraviolet scattering agent contained as component (B) in the emulsion composition of the present invention, an inorganic powder or white pigment that has a high refractive index and physically reflects and scatters ultraviolet light on the surface thereof can be used, and examples thereof include zinc oxide, titanium oxide, fine particle zinc oxide, fine particle titanium oxide, and the like.

In addition, a powder or pigment obtained by subjecting the above-mentioned inorganic powders such as zinc oxide and the like or white pigment to a surface treatment with an inorganic compound such as silica, aluminum oxide, zirconium oxide or the like, or an organic compound such as silicone compound, fluorine compound (perfluoroalkylphosphoric acid ester, perfluoroalkylphosphoric acid salt, perfluoroalkylsilane, etc.), silane coupling agent, acylated amino acid (N-stearoyl-L-glutamic acid, N-lauroyl-L-lysine, etc.), metal soap (aluminum stearate, etc.), amino acid and the like can also be used. As such surface-treated powder or surface-treated pigment, for example, silica-treated zinc oxide, silica-treated titanium oxide, silica-treated fine particle zinc oxide, silica-treated fine particle titanium oxide, silicone-treated zinc oxide, silicone-treated titanium oxide, silicone-treated fine particle zinc oxide, silicone-treated fine particle titanium oxide, N-stearoyl-L-glutamic acid-treated zinc oxide, N-stearoyl-L-glutamic acid-treated titanium oxide, N-stearoyl-L-glutamic acid-treated fine particle zinc oxide, N-stearoyl-L-glutamic acid-treated fine particle titanium oxide, N-lauroyl-L-lysine-treated zinc oxide, N-lauroyl-L-lysine-treated titanium oxide, N-lauroyl-L-lysine-treated fine particle zinc oxide, N-lauroyl-L-lysine-treated fine particle titanium oxide, aluminum stearate-treated zinc oxide, aluminum stearate-treated titanium oxide, aluminum stearate-treated fine particle zinc oxide, aluminum stearate-treated fine particle titanium oxide, and the like can also be used.

In the present invention, silica-treated zinc oxide, silica-treated titanium oxide, silica-treated fine particle zinc oxide, silica-treated fine particle titanium oxide, silicone-treated zinc oxide, silicone-treated titanium oxide, silicone-treated fine particle zinc oxide, silicone-treated fine particle titanium oxide, N-stearoyl-L-glutamic acid-treated zinc oxide, N-stearoyl-L-glutamic acid-treated titanium oxide, N-stearoyl-L-glutamic acid-treated fine particle zinc oxide, N-stearoyl-L-glutamic acid-treated fine particle titanium oxide, N-lauroyl-L-lysine-treated zinc oxide, N-lauroyl-L-lysine-treated titanium oxide, N-lauroyl-L-lysine-treated fine particle zinc oxide, N-lauroyl-L-lysine-treated fine particle titanium oxide and the like can be preferably used.

In the present invention, as component (B), one kind may be selected from the group consisting of the ultraviolet absorbers and ultraviolet scattering agents mentioned above and used alone, or two or more kinds thereof may be selected and used in combination.

From the aspect of imparting protective effect against medium wavelength ultraviolet rays (UVB) and long-wavelength ultraviolet rays (UVA), it is preferable to use one or more kinds of absorbers for medium wavelength ultraviolet rays (UVB) and one or more kinds of absorbers for long-wavelength ultraviolet rays (UVA) in combination, or use one or more kinds of absorbers for medium wavelength ultraviolet rays (UVB) and long-wavelength ultraviolet rays (UVA).

From the aspect of UV protective effect, it is preferable to use an ultraviolet absorber and a ultraviolet scattering agent in combination.

As the ultraviolet absorber and ultraviolet scattering agent, commercially available products provided by various companies can be used.

The content of (B) one or more selected from the group consisting of an ultraviolet absorber and an ultraviolet scattering agent in the emulsion composition of the present invention can be appropriately determined in consideration of the UV protective ability, legal regulations, and the like of the ultraviolet absorbers and ultraviolet scattering agents to be used. It is preferably 0.1 wt% to 25 wt%, more preferably 3 wt% to 25 wt%.

In the emulsion composition of the present invention, (C) one or more selected from the group consisting of divalent alcohol having 4 to 6 carbon atoms, fatty acid that is liquid at ordinary temperature, aliphatic alcohol that is liquid at ordinary temperature, an ester of straight chain saturated fatty acid and monovalent alcohol that is liquid at ordinary temperature, and an ester of aromatic carboxylic acid that is liquid at ordinary temperature is contained as an organic solvent to dissolve an oil gelling agent having an amide group to be contained as component (A).

As used herein, the "ordinary temperature" refers to the ordinary temperature defined in the Japanese Pharmacopoeia, i.e., 15°C to 25°C. In addition, "liquid" refers to a state with fluidity.

Examples of the divalent alcohol having 4 to 6 carbon atoms which is contained as component (C) in the emulsion composition of the present invention include 1,3-butanediol (1,3-butylene glycol), 1,2-pentanediol (pentylene glycol), 1,2-hexanediol and the like.

Examples of the fatty acid that is liquid at ordinary temperature which is contained as component (C) in the emulsion composition of the present invention include branched chain saturated fatty acids such as 14-methylpentadecanoic acid (isopalmitic acid), 16-methylheptadecanoic acid (isostearic acid), 3,7,11,15-tetramethylhexadecanoic acid (phytanic acid), and the like; unsaturated fatty acids such as 9-hexadecenoic acid (palmitoleic acid), cis-9-octadecenoic acid (oleic acid), and the like; and the like.

Examples of the aliphatic alcohol that is liquid at ordinary temperature which is contained as component (C) in the emulsion composition of the present invention include branched chain saturated aliphatic alcohols such as 2-hexyl-1-decanol, 16-methylheptadecanol (isostearyl alcohol), 2-octyl-1-dodecanol, 2-decyl-1-tetradecanol, and the like; unsaturated aliphatic alcohols such as cis-9-octadecene-1-ol (oleyl alcohol) and the like; and the like.

Examples of the ester of straight chain saturated fatty acid and monovalent alcohol that is liquid at ordinary temperature, which is contained as component (C) in the emulsion composition of the present invention, include esters of straight chain saturated fatty acid and lower secondary alcohol, such as isopropyl myristate, isopropyl palmitate, and the like.

Examples of the ester of aromatic carboxylic acid that is liquid at ordinary temperature include alkyl(C12-15) benzoate and the like.

From the aspect of solubility of an oil gelling agent having an amide group which is contained as component (A), divalent alcohol having 4 to 6 carbon atoms, fatty acid that is liquid at ordinary temperature, or aliphatic alcohol that is liquid at ordinary temperature is preferably used, divalent alcohol having 4 to 6 carbon atoms, branched chain saturated fatty acid, branched chain saturated aliphatic alcohol, unsaturated aliphatic alcohol, or the like is more preferably used, divalent alcohol having 4 to 6 carbon atoms, branched chain saturated fatty acid, branched chain saturated aliphatic alcohol, or the like is further preferably used, and branched chain saturated aliphatic alcohol is still more preferably used, as component (C) in the present invention.

As the branched chain saturated fatty acid, branched chain saturated fatty acid having 16 to 20 carbon atoms is preferably used, branched chain saturated fatty acid having 18 to 20 carbon atoms is more preferably used, branched chain saturated fatty acid having 18 carbon atoms is further preferably used.

As the branched chain saturated aliphatic alcohol, branched chain saturated aliphatic alcohol having 16 to 24 carbon atoms is preferably used, branched chain saturated aliphatic alcohol having 18 to 24 carbon atoms is more preferably used, and branched chain saturated aliphatic alcohol having 20 carbon atoms is further preferably used.

Specific examples of the component (C) used preferably include 1,3-butanediol (1,3-butylene glycol), 1,2-pentanediol (pentylene glycol), isostearic acid, isostearyl alcohol, 2-hexyl-1-decanol, 16-methylheptadecanol (isostearyl alcohol), 2-octyl-1-dodecanol, 2-decyl-1-tetradecanol, cis-9-octadecen-1-ol

(oleyl alcohol), and the like, examples of the component (C) used more preferably include 1,3-butanediol (1,3-butylene glycol), 1,2-pentanediol (pentylene glycol), isostearic acid, isostearyl alcohol, 2-octyl-1-dodecanol, 2-decyl-1-tetradecanol, and the like.

In the present invention, one kind of the above-mentioned divalent alcohol having 4 to 6 carbon atoms, fatty acid that is liquid at ordinary temperature, aliphatic alcohol that is liquid at ordinary temperature, an ester of straight chain saturated fatty acid and monovalent alcohol that is liquid at ordinary temperature, and an ester of aromatic carboxylic acid that is liquid at ordinary temperature may be selected and used alone, or two or more kinds may be selected and used in combination.

The divalent alcohol having 4 to 6 carbon atoms, fatty acid that is liquid at ordinary temperature, aliphatic alcohol that is liquid at ordinary temperature, an ester of straight chain saturated fatty acid and monovalent alcohol that is liquid at ordinary temperature, and an ester of aromatic carboxylic acid that is liquid at ordinary temperature may be produced by production methods known per se, and used, or commercially available products from various companies can also be used.

The content of (C) one or more selected from the group consisting of divalent alcohol having 4 to 6 carbon atoms, fatty acid that is liquid at ordinary temperature, aliphatic alcohol that is liquid at ordinary temperature, an ester of straight chain saturated fatty acid and monovalent alcohol that is liquid at ordinary temperature, and an ester of aromatic carboxylic acid that is liquid at ordinary temperature in the emulsion composition of the present invention is preferably 3 wt% to 50 wt%, more preferably 5 wt% to 30 wt%.

From the aspect of solubility of (A) oil gelling agent having an amide group, the content ratio of (A) oil gelling agent having an amide group and (C) one or more selected from the group consisting of divalent alcohol having 4 to 6 carbon atoms, fatty acid that is liquid at ordinary temperature, aliphatic alcohol that is liquid at ordinary temperature, an ester of straight chain saturated fatty acid and monovalent alcohol that is liquid at ordinary temperature, and an ester of aromatic carboxylic acid that is liquid at ordinary temperature [(A):(C)] is preferably 3:5 to 1:20, more preferably 1:3 to 1:19, further preferably 1:7 to 1:19, in weight ratios.

The one or more selected from the group consisting of divalent alcohol having 4 to 6 carbon atoms, fatty acid that is liquid at ordinary temperature, aliphatic alcohol that is liquid at ordinary temperature, an ester of straight chain saturated fatty acid and monovalent alcohol that is liquid at ordinary temperature, and an ester of aromatic carboxylic acid that is liquid at ordinary temperature, contained as the component (C) in the emulsion composition of the present invention is contained for the purpose of dissolving the (A) oil gelling agent having an amide group, and may also be contained for the purpose of dissolving, dispersing, etc. the general additives described below. For example, divalent alcohol having 4 to 6 carbon atoms may also be contained for the purpose of dissolving, dispersing, etc. hydrophilic additives, and fatty acid that is liquid at ordinary temperature, aliphatic alcohol that is liquid at ordinary temperature, an ester of straight chain saturated fatty acid and monovalent alcohol that is liquid at ordinary temperature, and an ester of aromatic carboxylic acid that is liquid at ordinary temperature may also be contained for the purpose of dissolving, dispersing, etc. lipophilic additives.

When one or more selected from the group consisting of divalent alcohol having 4 to 6 carbon atoms, fatty acid that is liquid at ordinary temperature, aliphatic alcohol that is liquid at ordinary temperature, an ester of straight chain saturated fatty acid and monovalent alcohol that is liquid at ordinary temperature, and an ester of aromatic carboxylic acid that is liquid at ordinary temperature, to be contained as component (C), is/are also contained for the purpose of dissolving, dispersing, etc. general additives, in addition to the purpose of dissolving (A) an oil gelling agent having an amide group, the content ratio of the above-mentioned (A) oil gelling agent having an amide group and (C) one or more selected from the group consisting of divalent alcohol having 4 to 6 carbon atoms, fatty acid that is liquid at ordinary temperature, aliphatic alcohol that is liquid at ordinary temperature, an ester of straight chain saturated fatty acid and monovalent alcohol that is liquid at ordinary temperature, and an ester of aromatic carboxylic acid that is liquid at ordinary temperature [(A):(C)] is calculated from the content of the component (A) and the content of the component (C) used to dissolve, disperse, etc. the component (A), from the total content of the component (C).

As the emulsifier contained as component (D) in the emulsion composition of the present invention, an emulsifier suitable for preparing an oil-in-water type emulsion composition or a water-in-oil type emulsion composition can be appropriately selected and used according to the types and contents of the oily phase components and aqueous phase components contained in the emulsion composition.

Examples include anionic surfactants such as higher fatty acid salts (potassium stearate, sodium stearate, etc.), alkyl ether carboxylates (sodium polyoxyethylene lauryl ether acetate, etc.), acyl lactates (sodium isostearoyl lactylate, etc.), N-acyl sarcosinates (sodium N-lauroyl sarcosinate, etc.), N-acyl glutamates (sodium N-lauroyl-L-glutamate, sodium N-stearoyl-L-glutamate, etc.), alkyl sulfates (sodium lauryl sulfate, etc.), and the like; amphoteric surfactants such as alkylaminopropionates (e.g., sodium β-laurylaminopropionate, etc.), N-acylaminoethyl-N-2-hydroxyethylpropionates (e.g., sodium N-cocoyl acylaminoethyl-N-2-hydroxyethylpropionate, etc.), lecithin, and the like; non-ionic surfactants such as polyoxyethylene alkyl ethers (polyoxyethylene cetyl ether, etc.), polyoxyethylene alkylphenyl ethers (polyoxyethylene octylphenyl ether, etc.), polyoxyethylene polyoxypropylene alkyl ethers (polyoxyethylene polyoxypropylene cetyl ether, etc.), polyoxyethylene fatty acid esters (polyethylene glycol monostearate, etc.), polyoxyethylene sorbitan fatty acid esters (polyoxyethylene sorbitan monolaurate, polyoxyethylene sorbitan monostearate, etc.), polyoxyethylene castor oil, polyoxyethylene hydrogenated castor oil, propylene glycol fatty acid esters (propylene glycol monostearate, etc.), glycerin fatty acid esters (glycerin monostearate, etc.), polyglycerin fatty acid esters (tetraglycerin monostearate, decaglycerin monostearate, etc.), sorbitan fatty acid esters (sorbitan monostearate, etc.), sucrose fatty acid esters (sucrose monopalmitate, sucrose monostearate, etc.), alkyl polyglycosides (coconut oil alkyl glucoside, etc.), polyether-modified silicones (polyoxyethylene-modified dimethyl polysiloxane, polyoxypropylene-modified dimethyl polysiloxane, polyoxyethylene-polyoxypropylene-modified dimethyl polysiloxane, etc.), and the like.

One or more kinds of emulsifiers mentioned above can be appropriately selected and used according to the property and the like of the emulsion composition to be prepared.

As the above-mentioned various emulsifiers, commercially available products provided by various companies can be used.

The content of the (D) emulsifier in the emulsion composition of the present invention can be appropriately set depending on the kinds and amounts of the oily phase components and aqueous phase components contained in the emulsion composition, the kind and the like of the emulsifier to be contained, and the like. It is generally 0.1 wt% to 10 wt%, preferably 0.5 wt% to 8 wt%, more preferably 1 wt% to 5 wt%.

From the aspects of emulsification stability and ease of emulsification, the content ratio of the (A) gelling agent having an amide group and the (D) emulsifier [(A):(D)] in the emulsion composition of the present invention is preferably 1:30 to 30:1, more preferably 1:25 to 25:1, further preferably 1:20 to 20:1, still more preferably 1:15 to 15:1, in weight ratio.

In the emulsion composition of the present invention, the water contained as component (E) can be water suitable for producing cosmetics, and for example, purified water such as distilled water and ion-exchanged water can be used.

The content of water (E) in the emulsion composition of the present invention can be set as an amount with respect to the total amount of the emulsion composition as 100 wt%.

The emulsion composition of the present invention preferably further contains (F) an oily component.

As the oily component contained as component (F) in the emulsion composition of the present invention, any component that is generally used as an oily base, an oily emollient component, or the like in cosmetics can be used without a particular limitation. Examples include animal and vegetable fats and oils such as olive oil, soybean oil, camellia oil, sesame oil, peanut oil, cocoa butter, beef tallow, and lard; waxes such as carnauba wax, beeswax, and jojoba oil; fatty alcohols other than the above-mentioned component (C), such as cetyl alcohol and stearyl alcohol; fatty acids other than the above-mentioned component (C), such as palmitic acid and stearic acid; hydrocarbons such as squalane, white petrolatum, liquid paraffin, ceresin, and microcrystalline wax; ethers such as glycerin mono 2-ethylhexyl ether, glycerin monocetyl ether, glycerin monostearyl ether, and glycerin monooleyl ether; esters other than the above-mentioned component (C), such as cetyl 2-ethylhexanoate, dimer dilinoleic acid (phytosteryl/isostearyl/cetyl/stearyl/behenyl), N-lauroylsarcosine isopropyl, N-lauroyl-L-glutamic acid di(cholesteryl/2-octyldodecyl), N-lauroyl-L-glutamic acid di(cholesteryl/behenyl/2-octyldodecyl), N-lauroyl-L-glutamic acid di(phytosteryl/2-octyldodecyl), N-lauroyl-L-glutamic acid di(phytosteryl/behenyl/2-octyldodecyl), tri(caprylic/capric)glyceryl, glyceryl tristearate, glyceryl tribehenate, and the like; silicone oils such as octamethylcyclotetrasiloxane, decamethylcyclopentasiloxane, dimethylpolysiloxane, and methylphenylpolysiloxane; and the like.

in the present invention, one kind of the above-mentioned oily component may be selected and used alone, or two or more kinds may be selected and used in combination.

From the aspects of feel of use and the like of the emulsion composition of the present invention, hydrocarbons such as squalane, liquid paraffin, and microcrystalline wax; esters such as N-lauroyl sarcosine isopropyl, N-lauroyl-L-glutamic acid di(cholesteryl/2-octyldodecyl), N-lauroyl-L-glutamic acid di(cholesteryl/behenyl/2-octyldodecyl), N-lauroyl-L-glutamic acid di(phytosteryl/2-octyldodecyl), N-lauroyl-L-glutamic acid di(phytosteryl/behenyl/2-octyldodecyl), and tribehenate glyceryl; and the like are preferably used, and esters of N-acylamino acids such as N-lauroyl sarcosine isopropyl, N-lauroyl-L-glutamic acid di(cholesteryl/2-octyldodecyl), N-lauroyl-L-glutamic acid di(cholesteryl/behenyl/2-octyldodecyl), N-lauroyl-L-glutamic acid di(phytosteryl/2-octyldodecyl), N-lauroyl-L-glutamic acid di(phytosteryl/behenyl/2-octyldodecyl), and the like are more preferably used.

As the oily component mentioned above, commercially available products provided by various companies can be used.

The content of the oily component (F) in the emulsion composition of the present invention is preferably 0.1 wt% to 80 wt%, more preferably 1 wt% to 50 wt%.

As long as the characteristics of the present invention are not impaired, the emulsion composition of the present invention can contain general additives that are generally added to cosmetics.

Examples of the additive include polar organic solvents such as lower alcohols (ethanol, isopropanol, etc.); hydrophilic bases such as gelatin and polyethylene glycol; moisturizers such as propylene glycol, glycerin, maltitol, sodium pyrrolidonecarboxylate, and sodium hyaluronate; antiinflammatory agents such as allantoin, dipotassium glycyrrhizinate, and stearyl glycyrrhetinate; suspending agents such as propylene glycol alginate, dioctyl sodium sulfosuccinate, povidone, and the like; hydrophilic thickeners such as carboxyvinyl polymer, sodium polyacrylate, xanthan gum, carboxymethylcellulose, hydroxyethylcellulose, hydroxypropylcellulose, poly(vinyl alcohol) (partially saponified product), anhydrous magnesium sulfate, and the like; stabilizers such as disodium ethylenediaminetetraacetate, sorbitol, thymol, polyoxyethylene polyoxypropylene glycol, and the like; antioxidants such as ascorbic acid, sodium erythorbate, tocopheryl acetate, dibutyl hydroxytoluene and the like; preservatives such as sorbic acid, sodium dehydroacetate, methyl p-hydroxybenzoate, propyl p-hydroxybenzoate, butyl p-hydroxybenzoate, phenoxyethanol, and the like; pH adjusters such as hydrochloric acid, citric acid, sodium citrate, acetic acid, sodium acetate, potassium hydroxide, sodium hydroxide, sodium hydrogenphosphate, and the like; organic modified clay minerals such as dimethyl distearyl ammonium hectorite; film-forming agents such as eicosene-vinylpyrrolidone copolymer; extender pigments such as magnesium silicate, talc, mica, kaolin, and sericite; coloring pigments such as red iron oxide, yellow iron oxide, black iron oxide, ultramarine, ferric hexacyanoferrate, chromium oxide, and manganese violet; hydrophobized pigments of the above extender pigments and coloring pigments; organic powders such as nylon powder and N-lauroyl-L-lysine; coloring pigments such as gardenia pigment, safflower pigment, Blue No. 1, and Red No. 201; fragrances, and the like.

Where necessary, one or more kinds of the above-mentioned additives can be used.

The emulsion composition of the present invention may be either an oil-in-water type emulsion composition or a water-in-oil type emulsion composition. From the aspects of ease of application to the skin, spreadability, and the like, a liquid emulsion composition is adapted.

In the present invention, the "liquid emulsion composition" refers to an emulsion composition that exhibits fluidity, and includes emulsion compositions in a viscous state as long as they exhibit fluidity.

In addition, from the aspects of handleability when applied to the skin, spreadability on the skin, feel of use, and the like, it is preferable that the emulsion composition of the present invention has a certain degree of viscosity.

The viscosity of the emulsion composition of the present invention is preferably 300 mPa·s to 1,000 Pa·s, more preferably 300 mPa·s to 500 Pa·s, when measured at 25°C using a B-type viscometer.

In the emulsion composition of in the present invention, the (A) oil gelling agent having an amide group exhibits the effect of improving the ultraviolet protective effect of (B) one or more selected from the group consisting of an ultraviolet absorber and an ultraviolet scattering agent, i.e., the protective effect against medium wavelength ultraviolet rays (UVB) shown by the SPF value and/or the protective effect against long-wavelength ultraviolet rays (UVA) shown by the UVAPF value, and does not function as a gelling agent for oily substances such as oil-soluble ultraviolet absorbers, oily components, and the like.

Therefore, in the emulsion composition of in the present invention, the (A) oil gelling agent having an amide group does not form a three-dimensional network structure that solidifies oily substances such as oil-soluble ultraviolet absorbers, oily components, and the like, and it is considered that oily substances such as oil-soluble ultraviolet absorbers, oily components, and the like do not exist in a state of being encapsulated within the network structure.

The emulsion composition of the present invention can be produced in accordance with a general method for producing emulsion compositions, such as the agent-in-water method, the agent-in-oil method, the phase inversion emulsification method, the PIT emulsification method, the D-phase emulsification method, and the like.

That is, for example, when the emulsion composition of the present invention is an oil-in-water type emulsion composition, it can be produced by dissolving a hydrophilic emulsifier, which is selected as component (D), by adding to and mixing with (E) water, dissolving as necessary a hydrophilic additive by adding thereto and mixing therewith to prepare an aqueous phase, adding an oily phase which is prepared by mixing (B) an ultraviolet absorber with a lipophilic additive, which is added as necessary, and (F) an oily component, which is added as necessary, to the aqueous phase, followed by stirring and mixing, adding (B) an ultraviolet scattering agent and a powder component, which is added as necessary as an additive, followed by mixing and uniformly dispersing, adding (A) an oil gelling agent having an amide group which is dissolved in advance in (C) one or more selected from the group consisting of divalent alcohol having 4 to 6 carbon atoms, fatty acid that is liquid at ordinary temperature, aliphatic alcohol that is liquid at ordinary temperature, an ester of straight chain saturated fatty acid and monovalent alcohol that is liquid at ordinary temperature, and an ester of aromatic carboxylic acid that is liquid at ordinary temperature, followed by mixing until uniform.

When the phase inversion emulsification method is used, the emulsion composition can be produced by adding component (D) to the above-mentioned oily phase, adding the above-mentioned aqueous phase to an oily phase added component (D), followed by stirring and mixing, then adding and uniformly dispersing a powder component, and adding component (A) dissolved in component (C), followed by mixing.

In addition, when the emulsion composition of the present invention is a water-in-oil type emulsion composition, it can be produced by mixing a lipophilic emulsifier, which is selected as component (D), with (B) an ultraviolet absorber, (F) an oily component, which is added as necessary, and a lipophilic additive, which is added as necessary, to give an oily phase, adding an aqueous phase prepared by adding a hydrophilic additive to (E) water as necessary, followed by stirring and mixing, adding (B) an ultraviolet scattering agent and a powder component, which is added as necessary as an additive, followed by mixing and uniformly dispersing, adding (A) an oil gelling agent having an amide group which is dissolved in advance in (C) one or more selected from the group consisting of divalent alcohol having 4 to 6 carbon atoms, fatty acid that is liquid at ordinary temperature, aliphatic alcohol that is liquid at ordinary temperature, an ester of straight chain saturated fatty acid and monovalent alcohol that is liquid at ordinary temperature, and an ester of aromatic carboxylic acid that is liquid at ordinary temperature, followed by mixing until uniform.

The emulsion composition of the present invention can be provided as sunscreen cosmetics such as sunscreen lotion and the like, and makeup cosmetics such as liquid foundation and the like.

The emulsion composition of the present invention has high UV protective effect and is also superior in emulsification stability and feel of use.

In addition, the emulsion composition of the present invention can also be provided as a sunscreen preparation free of silicone-based materials or petroleum-based polymers.

The present invention also provides a method for improving a UV protective effect of an emulsion composition (hereinafter also to be referred to as "the improvement method of the present invention").

The improvement method of the present invention includes preparing an emulsion composition (1) containing (B) one or more selected from the group consisting of an ultraviolet absorber and an ultraviolet scattering agent, (D) an emulsifier, and (E) water, dissolving (A) an oil gelling agent having an amide group in (C) one or more selected from the group consisting of divalent alcohol having 4 to 6 carbon atoms, fatty acid that is liquid at ordinary temperature, aliphatic alcohol that is liquid at ordinary temperature, an ester of straight chain saturated fatty acid and monovalent alcohol that is liquid at ordinary temperature, and an ester of aromatic carboxylic acid that is liquid at ordinary temperature, adding the solution to the aforementioned emulsion composition (1), and mixing them to obtain a liquid emulsion composition (2).

The (A) oil gelling agent having an amide group, (B) one or more selected from the group consisting of an ultraviolet absorber and an ultraviolet scattering agent, (C) one or more selected from the group consisting of divalent alcohol having 4 to 6 carbon atoms, fatty acid that is liquid at ordinary temperature, aliphatic alcohol that is liquid at ordinary temperature, an ester of straight chain saturated fatty acid and monovalent alcohol that is liquid at ordinary temperature, and an ester of aromatic carboxylic acid that is liquid at ordinary temperature, (D) emulsifier and (E) water are as described above for the emulsion composition of the present invention.

In addition, the content of each component relative to the total amount of the finally prepared liquid emulsion composition (2), the content ratio of component (A) and component (C), and the content ratio of component (A) and component (D) are also as described above for the emulsion composition of the present invention.

In the improvement method of the present invention, the emulsion composition (1) preferably further contains (F) an oily component.

The (F) oily component is as described above for the emulsion composition of the present invention, and the content of component (F) relative to the total amount of the finally prepared liquid emulsion composition (2) is also as described above for the emulsion composition of the present invention.

In the improvement method of the present invention, emulsion composition (1) can further contain general additives blended in cosmetics. Such general additives are also as described above for the emulsion composition of the present invention.

In the improvement method of the present invention, the emulsion composition (1) can be prepared according to a general production method of an oil-in-water type emulsion composition or water-in-oil type emulsion composition, and the detail of the preparation method is as described above for the method for producing the emulsion composition of the present invention.

In the improvement method of the present invention, the liquid emulsion composition (2) is prepared as a liquid oil-in-water type emulsion composition or a liquid water-in-oil type emulsion composition. The "liquid emulsion composition" is as described above for the emulsion composition of the present invention, and the viscosity thereof is also as described above for the emulsion composition of the present invention.

By the improvement method of the present invention, a UV protective effect of the finally-prepared liquid emulsion composition (2) is improved.

The liquid emulsion composition (2) finally prepared by the improvement method of the present invention can be provided as sunscreen cosmetics such as sunscreen lotion and the like, and makeup cosmetics such as liquid foundation and the like, and can also be provided as a sunscreen preparation free of silicone-based materials or petroleum-based polymers.

Therefore, the present invention also relates to the use of (A) an oil gelling agent having an amide group for improving a UV protective effect in a liquid emulsion composition containing (B) one or more selected from the group consisting of an ultraviolet absorber and an ultraviolet scattering agent, (C) one or more selected from the group consisting of divalent alcohol having 4 to 6 carbon atoms, fatty acid that is liquid at ordinary temperature, aliphatic alcohol that is liquid at ordinary temperature, an ester of straight chain saturated fatty acid and monovalent alcohol that is liquid at ordinary temperature, and an ester of aromatic carboxylic acid that is liquid at ordinary temperature, (D) an emulsifier, and (E) water, or the emulsion composition further containing (F) an oily component (hereinafter also to be referred to as "the use of the present invention").

In the use of the present invention, the (A) oil gelling agent having an amide group is added by dissolving in the above-mentioned component (C) in the preparation of the liquid emulsion composition.

### [Example]

The present invention is explained in more detail in the following by referring to Examples.

### [Examples 1 - 11, Reference Example 1, Comparative Examples 1 - 10] Oil-in-water type emulsion composition

According to the formulations shown in Tables 1, 2, component (B) was mixed with component (F) and dissolved with stirring to form an oily phase. Meanwhile, component (D) and a hydrophilic additive were added to component (E) and dissolved with stirring to form an aqueous phase. Then, while stirring the aqueous phase at 400 rpm, the oil phase was added, mixed, and emulsified, and powder was added and uniformly dispersed. Furthermore, a solution of component (A) dissolved in component (C) in advance was added, and the mixture was mixed and stirred to form the oil-in-water type emulsion compositions of Examples 1 - 11, Reference Example 1, and Comparative Examples 1 - 10.

As shown below, the appearance of each of the oil-in-water type emulsion compositions of Examples 1 - 11, Reference Example 1 and Comparative Examples 1 - 10 was observed and the UV protective effect was evaluated. The evaluation results are shown together in Tables 1, 2.

### (1) Appearance observation

The appearance of each of the oil-in-water type emulsion compositions of Examples, Reference Example and Comparative Examples was observed, and those that had a liquid appearance and flowed when tilted at 25°C for one minute or more were rated as "A", those that showed partial gelation were rated as "B", and those that showed a uniform gel-like state were rated as "C".

### (2) Evaluation of UV protective effect

Each (32 mg) of the oil-in-water type emulsion compositions of the Examples, Reference Example, and Comparative Examples was weighed out onto a 50 mm x 50 mm polymethyl methacrylate plate and uniformly applied with the ball of a finger, after which the SPF value and UVAPF value were measured using an SPF analyzer (UV2000S) manufactured by Labsphere. Measurement using the SPF analyzer was performed at 10 points for each emulsion composition applied to the polymethyl methacrylate plate, and the average values were calculated and respectively taken as the SPF value and UVAPF value.

**[Table 1]**

| component | | supplier | trade name | Ex. 1 | Ex. 2 | Ex. 3 | Ex. 4 | Ref. Ex. 1 | Ex. 5 | Ex. 6 | Ex. 7 | Ex. 8 | Ex. 9 | Ex. 10 | Ex. 11 |
|---|---|---|---|---|---|---|---|---|---|---|---|---|---|---|---|
| (A) | N-lauroyl-L-glutamic acid dibutylamide | Ajinomoto Co., Inc. | GP-1 | 0.24 | 0.6 | 0.24 | 0.6 | 0.24 | 0.6 | 0.24 | 0.6 | 0.6 | 0.6 | 1.2 | 1.8 |
| | N-2-ethylhexanoyl-L-glutamic acid dibutylamide | Ajinomoto Co., Inc. | EB-21 | 0.16 | 0.4 | 0.16 | 0.4 | 0.16 | 0.4 | 0.16 | 0.4 | 0.4 | 0.4 | 0.8 | 1.2 |
| (B) | 4-t-butyl-4'-methoxydi-benzovlmethane | DSM | Parsol 1789 | 3.0 | 3.0 | | | | | | | 3.0 | 3.0 | 3.0 | 3.0 |
| | 2-cyano-3, 3-diphenyl-2-propenoic acid 2-ethylhexyl ester | DSM | Parsol 340 | 10.0 | 10.0 | | | | | | | 10.0 | 10.0 | 10.0 | 10.0 |
| | 2-ethylhexyl salicylate | DSM | Parsol EHS | 5.0 | 5.0 | | | | | | | 5.0 | 5.0 | 5.0 | 5.0 |
| | bis-ethylhexyloxyphenol methoxyphenyltriazine | BASF | Tinosorb (R) S | | | 3.0 | 3.0 | | | | | | | | |
| | 2-[4-(diethyl-amino)-2-hydroxybenzoyl]-benzoic acid hexyl ester | BASF | Uvinul A | | | | | 10.0 | 10.0 | | | | | | |
| | 2-ethylhexyl p-methoxycinnamate | Ashland Japan | ESCALOL 557 | | | | | | | 14.0 | 14.0 | | | | |
| (C) | 2-octyl-1-dodecanol | KOKYU ALCOHOL KOGYO CO., LTD. | RISONOL 20SP | 7.6 | 7.0 | 7.6 | 7.0 | 7.6 | 7.0 | 7.6 | 7.0 | | | | |
| | 1,3-butylene glycol | Daicel Corporation | 1.3BG UK | | | | | | | | | 7.0 | | | |
| | isostearic acid | KOKYU ALCOHOL KOGYO CO., LTD. | isostearic acid EX | | | | | | | | | | 7.0 | 6.0 | 5.0 |
| (F) | N-lauroyl-L-glutamic acid di(phytosteryl/ 2-octvldodecvl) | Ajinomoto Co., Inc. | ELDEW PS-203R | 3.0 | 3.0 | 3.0 | 3.0 | 3.0 | 3.0 | 3.0 | 3.0 | 3.0 | 3.0 | 3.0 | 3.0 |
| | N-lauroylsarcosine isopropyl | Ajinomoto Co., Inc. | ELDEW SL-205 | 7.0 | 7.0 | 7.0 | 7.0 | 7.0 | 7.0 | 7.0 | 7.0 | 7.0 | 7.0 | 7.0 | 7.0 |
| powder | N-lauroyl-L-lysine | Ajinomoto Co., Inc. | Amihope LL | 5.0 | 5.0 | 5.0 | 5.0 | 5.0 | 5.0 | 5.0 | 5.0 | 5.0 | 5.0 | 5.0 | 5.0 |
| (D) | sodium N-stearoyl-L-glutamate | Ajinomoto Co., Inc. | AMISOFT HS-11P | 2.6 | 2.6 | 2.6 | 2.6 | 2.6 | 2.6 | 2.6 | 2.6 | 2.6 | 2.6 | 2.6 | 2.6 |
| hydrophilic additive | glycerin | Kao Corporation | concentrated glycerin | 2.0 | 2.0 | 2.0 | 2.0 | 2.0 | 2.0 | 2.0 | 2.0 | 2.0 | 2.0 | 2.0 | 2.0 |
| | propylene glycol | ADEKA | ADEKA PG | 2.0 | 2.0 | 2.0 | 2.0 | 2.0 | 2.0 | 2.0 | 2.0 | 2.0 | 2.0 | 2.0 | 2.0 |
| | xanthan gum | CP Kelco | KELTROL CG-T | 0.5 | 0.5 | 0.5 | 0.5 | 0.5 | 0.5 | 0.5 | 0.5 | 0.5 | 0.5 | 0.5 | 0.5 |
| | phenoxyethanol | Yokkaichi Chemical Co., Ltd. | phenoxyethanol S | 0.3 | 0.3 | 0.3 | 0.3 | 0.3 | 0.3 | 0.3 | 0.3 | 0.3 | 0.3 | 0.3 | 0.3 |
| (E) | ion exchange water | prepared using ion exchange resin | | 51.6 | 51.6 | 66.6 | 66.6 | 59.6 | 59.6 | 55.6 | 55.6 | 51.6 | 51.6 | 51.6 | 51.6 |
| total | | | | 100.0 | 100.0 | 100.0 | 100.0 | 100.0 | 100.0 | 100.0 | 100.0 | 100.0 | 100.0 | 100.0 | 100.0 |
| (C) / (A) | | | | 19.0 | 7.0 | 19.0 | 7.0 | 19.0 | 7.0 | 19.0 | 7.0 | 7.0 | 7.0 | 3.0 | 1.7 |
| evaluation item | appearance observation results | | | A | A | A | A | A | A | A | A | A | A | A | A |
| | SPF value | | | 14.87 | 37.24 | 3.56 | 5.46 | - | - | 14.26 | 17.79 | 11.07 | 6.44 | 9.41 | 11.87 |
| | UVAPF value | | | 11.93 | 27.97 | 0.33 | 0.44 | 0.94 | 7.34 | - | - | 8.34 | 5.87 | 7.88 | 10.55 |

| | | | | | | | | | | | | | | | |
|---|---|---|---|---|---|---|---|---|---|---|---|---|---|---|---|
| * In the Table, the numerical value corresponding to each component indicates the content (wt%) of the component, and the blank corresponding to each component indicates that the component is not contained. ** In the Table, "-" in the column corresponding to the SPF value and UVAPF value indicates that the UV protective effect was not evaluated. | | | | | | | | | | | | | | | |

**[Table 2]**

| component | | supplier | trade name | Comp. Ex. 1 | Comp. Ex. 2 | Comp. Ex. 3 | Comp. Ex. 4 | Comp. Ex. 5 | Comp. Ex. 6 | Comp. Ex. 7 | Comp. Ex. 8 | Comp. Ex. 9 | Comp. Ex. 10 |
|---|---|---|---|---|---|---|---|---|---|---|---|---|---|
| (A) | N-lauroyl-L-glutamic acid dibutylamide | Ajinomoto Co., Inc. | GP-1 | | | | | 1.2 | | 1.2 | 1.8 | | 3.0 |
| | N-2-ethylhexanoyl-L-glutamic acid dibutylamide | Ajinomoto Co., Inc. | EB-21 | | | | | 0.8 | | 0.8 | 1.2 | | 2.0 |
| (B) | 4-t-butyl-4'-methoxydibenzoylmethane | DSM | Parsol 1789 | 3.0 | | | | 3.0 | 3.0 | 3.0 | 3.0 | 3.0 | 3.0 |
| | 2-cyano-3, 3-diphenyl-2-propenoic acid 2-ethylhexyl ester | DSM | Parsol 340 | 10.0 | | | | 10.0 | 10.0 | 10.0 | 10.0 | 10.0 | 10.0 |
| | 2-ethylhexyl salicylate | DSM | Parsol EHS | 5.0 | | | | 5.0 | 5.0 | 5.0 | 5.0 | 5.0 | 5.0 |
| | bis-ethylhexyloxyphenol methoxyphenyltriazine | BASF | Tinosorb^{®} S | | 3.0 | | | | | | | | |
| | 2-[4-(diethylamino)-2-hydroxybenzoyl]-benzoic acid hexyl ester | BASF | Uvinul A | | | 10.0 | | | | | | | |
| | 2-ethylhexyl p-methoxycinnamate | Ashland Japan | ESCALOL 557 | | | | 14.0 | | | | | | |
| (C) | 2-octyl-1-dodecanol | KOKYU ALCOHOL KOGYO CO., LTD. | RISONOL 20SP | 8.0 | 8.0 | 8.0 | 8.0 | 6.0 | | | | | |
| | 1,3-butylene glycol | Daicel Corporation | 1.3BG UK | | | | | | 8.0 | 6.0 | 5.0 | | |
| | isostearic acid | KOKYU ALCOHOL KOGYO CO., LTD. | isostearic acid EX | | | | | | | | | 8.0 | 3.0 |
| (F) | N-lauroyl-L-glutamic acid di(phytosteryl/2-octyldodecyl) | Ajinomoto Co., Inc. | ELDEW PS-203R | 3.0 | 3.0 | 3.0 | 3.0 | 3.0 | 3.0 | 3.0 | 3.0 | 3.0 | 3.0 |
| | N-lauroylsarcosine isopropyl | Ajinomoto Co., Inc. | ELDEW SL-205 | 7.0 | 7.0 | 7.0 | 7.0 | 7.0 | 7.0 | 7.0 | 7.0 | 7.0 | 7.0 |
| powder | N-lauroyl-L-lysine | Ajinomoto Co., Inc. | Amihope LL | 5.0 | 5.0 | 5.0 | 5.0 | 5.0 | 5.0 | 5.0 | 5.0 | 5.0 | 5.0 |
| (D) | sodium N-stearoyl-L-glutamate | Ajinomoto Co., Inc. | AMISOFT HS-11P | 2.6 | 2.6 | 2.6 | 2.6 | 2.6 | 2.6 | 2.6 | 2.6 | 2.6 | 2.6 |
| hydrophilic additive | glycerin | Kao Corporation | concentrated qlvcerin | 2.0 | 2.0 | 2.0 | 2.0 | 2.0 | 2.0 | 2.0 | 2.0 | 2.0 | 2.0 |
| | propylene glycol | ADEKA CORPORATION | ADEKA PG | 2.0 | 2.0 | 2.0 | 2.0 | 2.0 | 2.0 | 2.0 | 2.0 | 2.0 | 2.0 |
| | xanthan gum | CP Kelco | KELTROL CG-T | 0.5 | 0.5 | 0.5 | 0.5 | 0.5 | 0.5 | 0.5 | 0.5 | 0.5 | 0.5 |
| | phenoxyethanol | Yokkaichi Chemical Co., Ltd. | phenoxyethanol S | 0.3 | 0.3 | 0.3 | 0.3 | 0.3 | 0.3 | 0.3 | 0.3 | 0.3 | 0.3 |
| (E) | ion exchange water | prepared using ion exchange resin | | 51.6 | 66.6 | 59.6 | 55.6 | 51.6 | 51.6 | 51.6 | 51.6 | 51.6 | 51.6 |
| total | | | | 100.0 | 100.0 | 100.0 | 100.0 | 100.0 | 100.0 | 100.0 | 100.0 | 100.0 | 100.0 |
| (C) / (A) | | | | - | - | - | - | 3.0 | - | 3.0 | 1.7 | - | 0.6 |
| evaluation item | appearance observation results | | | A | A | A | A | B | A | B | C | A | C |
| | SPF value | | | 10.33 | 2.44 | - | 7.9 | - | 3.14 | - | - | 5.69 | - |
| | UVAPF value | | | 9.01 | 0.2 | 1.17 | - | - | 2.87 | - | - | 5.32 | - |

| | | | | | | | | | | | | | |
|---|---|---|---|---|---|---|---|---|---|---|---|---|---|
| * In the Table, the numerical value corresponding to each component indicates the content (wt%) of the component, and the blank corresponding to each component indicates that the component is not contained. ** In the Table, "-" in the column corresponding to the (C)/(A) value indicates that the numerical value cannot be calculated because component (A) is not contained. *** In the Table, "-" in the column corresponding to the SPF value and UVAPF value indicates that the UV protective effect was not evaluated. | | | | | | | | | | | | | |

As shown in Tables 1, 2, when 4-t-butyl-4'-methoxydibenzoylmethane (3.0 wt%), 2-cyano-3,3-diphenyl-2-propenoic acid 2-ethylhexyl ester (octocrylene) (10.0 wt%) and 2-ethylhexyl salicylate (5.0 wt%) were contained as component (B), in the oil-in-water type emulsion compositions (Examples 1, 2) prepared by adding N-lauroyl-L-glutamic acid dibutylamide and N-2-ethylhexanoyl-L-glutamic acid dibutylamide as component (A) dissolved in 2-octyl-1-dodecanol as component (C), an improvement in the SPF value and the UVAPF value was found depending on the content of component (A), compared to the oil-in-water type emulsion composition (Comparative Example 1) not containing component (A).

In the oil-in-water type emulsion compositions (Example 8, Example 9) prepared by using, in Example 2, 1,3-butylene glycol and isostearic acid instead of 2-octyl-1-dodecanol, respectively as component (C), and in the oil-in-water type emulsion compositions (Examples 10, 11) prepared by setting, in Example 9, the total content of component (A) to 2.0 wt% and 3.0 wt%, respectively, an improvement in the SPF value and the UVAPF value was found compared to the oil-in-water type emulsion composition (Comparative Examples 6, 9) not containing component (A). In the oil-in-water type emulsion compositions of Examples 9 - 11, an improvement in the SPF value and the UVAPF value was found in a component (A) content-dependent manner.

When 2-octyl-1-dodecanol was used as component (C), the highest improvement was found in the SPF value and the UVAPF value.

In addition, when bis-ethylhexyloxyphenol methoxyphenyltriazine (3.0 wt%) was contained as component (B), in the oil-in-water type emulsion composition (Examples 3 and 4) prepared by adding N-lauroyl-L-glutamic acid dibutylamide and N-2-ethylhexanoyl-L-glutamic acid dibutylamide as component (A) dissolved in 2-octyl-1-dodecanol as component (C), an improvement in the SPF value and the UVAPF value was found depending on the content of component (A), compared to the oil-in-water type emulsion composition (Comparative Example 2) not containing component (A).

When 2-[4-(diethylamino)-2-hydroxybenzoyl]benzoic acid hexyl ester (10.0 wt%) was contained as component (B), in the oil-in-water type emulsion composition (Example 5) prepared by adding 1.0 wt% in total of N-lauroyl-L-glutamic acid dibutylamide and N-2-ethylhexanoyl-L-glutamic acid dibutylamide as component (A) dissolved in 2-octyl-1-dodecanol as component (C), an improvement in the UVAPF value was found compared to the oil-in-water type emulsion composition (Comparative Example 3) not containing component (A). The oil-in-water type emulsion composition (Reference Example 1) with the total content of 0.4 wt% of the aforementioned component (A) did not show an improvement in the UVAPF value compared to Comparative Example 3.

When 2-ethylhexyl p-methoxycinnamate (14.0 wt%) was contained as component (B), in the oil-in-water type emulsion compositions (Examples 6, 7) prepared by adding N-lauroyl-L-glutamic acid dibutylamide and N-2-ethylhexanoyl-L-glutamic acid dibutylamide as component (A) dissolved in 2-octyl-1-dodecanol as component (C), an improvement in the SPF value was found depending on the content of component (A), compared to the oil-in-water type emulsion composition (Comparative Example 4) not containing component (A).

In the oil-in-water type emulsion compositions (Comparative Examples 5, 7) in which, in each of Examples 2 and 8, the contents of N-lauroyl-L-glutamic acid dibutylamide and N-2-ethylhexanoyl-L-glutamic acid dibutylamide were doubled (total content of component (A)=2.0 wt%), partial gel formation was observed.

In addition, the oil-in-water type emulsion composition (Comparative Example 8) in which, in Example 8, the contents of N-lauroyl-L-glutamic acid dibutylamide and N-2-ethylhexanoyl-L-glutamic acid dibutylamide were each tripled (total content of component (A)=3.0 wt%) and the oil-in-water type emulsion composition (Comparative Example 10) in which, in Example 9, the contents of N-lauroyl-L-glutamic acid dibutylamide and N-2-ethylhexanoyl-L-glutamic acid dibutylamide were each made fivefold (total content of component (A)=5.0 wt%) showed a gel-like appearance.

### [Examples 12 - 14, Comparative Example 11] Water-in-oil type emulsion composition

According to the formulations shown in Table 3, component (D) and a lipophilic additive were added to component (F), mixed, and dissolved with stirring to form an oily phase. Meanwhile, a hydrophilic additive was added to component (E) and dissolved with stirring to form an aqueous phase. Then, while stirring the oily phase at 400 rpm, the aforementioned aqueous phase was added, mixed, and emulsified, and component (B) and powder were added and uniformly dispersed. Furthermore, a solution of component (A) dissolved in component (C) in advance was added, and the mixture was mixed and stirred to make same homogeneous to give the water-in-oil type emulsion compositions of Examples 12 - 14 and Comparative Example 11.

In the same manner as for each of the oil-in-water emulsion compositions of the above-mentioned Example 1 - 11, Reference Example 1 and Comparative Example 1 - 10, the appearance of each of the water-in-oil type emulsion compositions of Examples 12 - 14 and Comparative Example 11 was observed and the SPF value and UVAPF value were measured. The results are also shown in Table 3.

**[Table 3]**

| component | | supplier | trade name | Ex. 12 | Ex. 13 | Ex. 14 | Comp. Ex. 11 |
|---|---|---|---|---|---|---|---|
| (A) | N-lauroyl-L-glutamic acid dibutylamide | Ajinomoto Co., Inc. | GP-1 | 0.24 | 0.6 | | |
| | N-2-ethylhexanoyl-L-glutamic acid dibutvlamide | Ajinomoto Co., Inc. | EB-21 | 0.16 | 0.4 | 1.0 | |
| (B) | titanium oxide | TAYCA CORPORATION | MT-100Z | 5.0 | 5.0 | 5.0 | 5.0 |
| | zinc oxide | TAYCA CORPORATION | MZY-303S | 10.0 | 10.0 | 10.0 | 10.0 |
| (C) | 2-octyl-1-dodecanol | KOKYU ALCOHOL KOGYO CO., LTD. | RISONOL 20SP | 7.6 | 7.0 | 7.0 | 8.0 |
| (F) | methylpolysiloxane | Shin-Etsu Chemical Co., Ltd. | KF-96A | 10.0 | 10.0 | 10.0 | 10.0 |
| | N-lauroyl-L-glutamic acid di(phytosteryl/behenyl/ 2-octyldodecyl) | Ajinomoto Co., Inc. | PS-304 | 1.0 | 1.0 | 1.0 | 1.0 |
| | decamethylcyclopentasiloxane | Dow Toray Co., Ltd. | SH-245 | 15.0 | 15.0 | 15.0 | 15.0 |
| lipophilic additive | tocopheryl acetate | TAMA BIOCHEMICAL CO., LTD. | acetic acid d-α-tocopherol | 0.1 | 0.1 | 0.1 | 0.1 |
| powder | N-lauroyl-L-lysine | Ajinomoto Co., Inc. | AMIHOPE LL | 3.0 | 3.0 | 3.0 | 3.0 |
| (D) | polyether-modified silicone | Shin-Etsu Chemical Co., Ltd. | KF6028 | 2.0 | 2.0 | 2.0 | 2.0 |
| hydrophilic additive | anhydrous magnesium sulfate | FUJIFILM Wako Pure Chemical Corporation | anhydrous magnesium sulfate reagent grade | 0.5 | 0.5 | 0.5 | 0.5 |
| | sodium pyrrolidonecarboxylate | Ajinomoto Co., Inc. | AJIDEW NL-50 | 0.5 | 0.5 | 0.5 | 0.5 |
| | glycerin | Kao Corporation | concentrated glycerin | 5.0 | 5.0 | 5.0 | 5.0 |
| | hydroxyethylcellulose | The Dow Chemical Company | CellosizeQP-30000H | 0.1 | 0.1 | 0.1 | 0.1 |
| | phenoxyethanol | Yokkaichi Chemical Co., Ltd. | phenoxyethanol S | 0.3 | 0.3 | 0.3 | 0.3 |
| (E) | purified water | prepared using ion exchange resin | | 39.5 | 39.5 | 39.5 | 39.5 |
| total | | | | 100.0 | 100.0 | 100.0 | 100.0 |
| (C) / (A) | | | | 19.0 | 7.0 | 7.0 | - |
| evaluation item | appearance observation results | | | A | A | A | A |
| | SPF value | | | 175.92 | 362.51 | 213.13 | 124.73 |
| | UVAPF value | | | 27.87 | 42.05 | 31.02 | 26.37 |

| | | | | | | | |
|---|---|---|---|---|---|---|---|
| * In the Table, the numerical value corresponding to each component indicates the content (wt%) of the component, and the blank corresponding to each component indicates that the component is not contained. ** In the Table, "-" in the column corresponding to the (C)/(A) value indicates that the numerical value cannot be calculated because component (A) is not contained. | | | | | | | |

As shown in Table 3, also in the water-in-oil type emulsion compositions containing an ultraviolet scattering agent as component (B), when component (A) dissolved in component (C) was contained (Examples 12 to 14), an improvement in the SPF value and UVAPF value was found depending on the amount of component (A), compared to when component (A) was not contained (Comparative Example 11).

In addition, when the total amount of N-acylamino acid dialkylamide contained as component (A) was the same, the water-in-oil type emulsion composition of Example 13, which contained N-lauroyl-L-glutamic acid dibutylamide and N-2-ethylhexanoyl-L-glutamic acid dibutylamide as component (A), showed a higher SPF value and a higher UVAPF value compared to the water-in-oil type emulsion composition of Example 14, which contained only N-2-ethylhexanoyl-L-glutamic acid dibutylamide. These results suggest that, from the aspect of improving the UV protective effect, it is preferable to use N-lauroyl-L-glutamic acid dibutylamide and N-2-ethylhexanoyl-L-glutamic acid dibutylamide in combination as component (A).

### [Example 15, Comparative Example 12] Silicone-free water-in-oil type liquid foundation

According to the formulation shown in Table 4 and using the following method, silicone-free water-in-oil type liquid foundations were prepared as the water-in-oil type emulsion compositions of Example 15 and Comparative Example 12.

### <Preparation method>

(1) Component (d1), component (f1), and the lipophilic additive in Table 4 were mixed and heated to 75°C until uniform. Component (b1), (d2), and the powder component in Table 4 were mixed and processed three times in a roll mill, and added to the aforementioned mixture, followed by mixing in a homo mixer (3,000 rpm, 5 min).
(2) Component (b2) in Table 4 was mixed with component (f2) and dissolved at room temperature. This was then added to (1) and mixed to give the oily phase.
(3) Component (c2) and the hydrophilic additive in Table 4 were added to (E), mixed, heated to 75°C until uniform, and used as the aqueous phase.
(4) The above-mentioned aqueous phase (3) was gradually added to the oily phase of the above-mentioned (2), and mixed in a homo mixer (3,000 rpm, 5 min) to emulsify.
(5) Component (A) in Table 4 was mixed with component (c1), dissolved by heating to 110°C in an oil bath, added to the above-mentioned (4) while still heating to 75°C and mixed, and then cooled to 40°C with stirring.

The appearance of each of the water-in-oil type liquid foundations of Example 15 and Comparative Example 12 was observed and the SPF value and the UVAPF value were measured in the same manner as for each of the oil-in-water type emulsion compositions of the above-mentioned Examples 1 to 11, Reference Example 1 and Comparative Examples 1 to 10. The results are also shown in Table 4.

**[Table 4]**

| component | | | supplier | trade name | Ex. 15 | Comp. Ex. 12 |
|---|---|---|---|---|---|---|
| (A) | | N-lauroyl-L-glutamic acid dibutylamide | Ajinomoto Co., Inc. | GP-1 | 0.6 | |
| | | N-2-ethylhexanoyl-L-glutamic acid dibutylamide | Ajinomoto Co., Inc. | EB-21 | 0.4 | |
| (B) | (b1) ultraviolet scattering agent | aluminum hydroxide and disodium N-stearoyl-L-glutamate-treated titanium dioxide | Miyoshi Kasei, Inc. | NAI-titanium CR-50 (100%) | 6.0 | 6.0 |
| | (b2) ultraviolet absorber | 4-t-butyl-4'-methoxydibenzoylmethane | DSM | Parsol 1789 | 3.0 | 3.0 |
| | | 2-cyano-3,3-diphenyl-2-propenoic acid 2-ethylhexyl ester | DSM | Parsol 340 | 10.0 | 10.0 |
| | | 2-ethylhexyl salicylate | DSM | Parsol EHS | 5.0 | 5.0 |
| (C) | (c1) | 1,3-butylene glycol | Daicel Corporation | 1,3 BG UK | 7.0 | 8.0 |
| | (c2) | 1,3-butylene glycol | Daicel Corporation | 1,3 BG UK | 6.0 | 6.0 |
| (F) | (f1) | isododecane | Presspers Corporation | Permethyl 99A | 5.4 | 5.4 |
| | | jojoba oil | Croda International Plc | Cropure JOJOBA | 2.0 | 2.0 |
| | | squalane | Maruha Nichiro Corporation | Squalane | 2.0 | 2.0 |
| | | N-lauroyl-L-glutamic acid di(phytosteryl/2-octyldodecyl) | Ajinomoto Co., Inc. | ELDEW PS-203 | 1.3 | 1.3 |
| | (f2) | isopropyl N-lauroyl sarcosinate | Ajinomoto Co., Inc. | ELDEW SL-205 | 7.0 | 7.0 |
| lipophilic additive | | dimethyl distearyl ammonium hectorite, isododecane, propylene carbonate | Elementis Plc | Bentone Gel ISD V | 2.7 | 2.7 |
| | | eicosene-vinylpyrrolidone copolymer | Givaudan S.A. | Unimer U-15 | 0.7 | 0.7 |
| powder | | disodium N-lauroyl-L-glutamate and aluminum hydroxide-treated red iron oxide | Miyoshi Kasei, Inc. | NAI-Red R-516PSR-516PSR-516PS (100%) | 0.2 | 0.2 |
| | | disodium N-lauroyl-L-glutamate and aluminum hydroxide-treated yellow iron oxide | Miyoshi Kasei, Inc. | NAI-Yellow LL-100P (100%) | 1. 0 | 1. 0 |
| | | disodium N-lauroyl-L-glutamate and aluminum hydroxide-treated black iron oxide | Miyoshi Kasei, Inc. | NAI-Black BL-100P (100%) | 0.1 | 0.1 |
| (D) | (d1) | diglyceryl monooleate, polyhydroxystearic acid, diglyceryl monostearate | Innovacos Corporation | Polyaquol OS2 | 1.3 | 1.3 |
| | | tetraglyceryl monooleate, polyhydroxystearic acid, hexaglyceryl monooleate | Innovacos Corporation | Polyaquol VO4 | 0.7 | 0.7 |
| | | condensed ricinoleic acid hexaglyceryl | Nikko Chemicals Co., Ltd. | Nikkol Hexaglyn PR-15 | 0.7 | 0.7 |
| | (d2) | diglyceryl monooleate, polyhydroxystearic acid, diglyceryl monostearate | Innovacos Corporation | Polyaquol OS2 | 0.7 | 0.7 |
| | | diglyceryl triisostearate | The Nisshin OilliO Group, Ltd. | Cosmol 43V | 3.4 | 3.4 |
| hydrophilic additive | | phenoxyethanol | Yokkaichi Chemical Co., Ltd. | phenoxyethanol S | 0.3 | 0.3 |
| | | maltitol | Tokyo Chemical Industry Co., Ltd. | Maltitol Amalti syrup | 2.0 | 2.0 |
| (E) | | purified water | prepared using ion exchange resin | | 30.5 | 30.5 |
| total | | | | | 100.0 | 100.0 |
| (c1) / (A) | | | | | 7.0 | - |
| evaluation item | | appearance observation results | | | A | A |
| | | SPF value | | | 196.02 | 117.20 |
| | | UVAPF value | | | 96.98 | 69.65 |

| | | | | | | |
|---|---|---|---|---|---|---|
| * In the Table, the numerical value corresponding to each component indicates the content (wt%) of the component, and the blank corresponding to each component indicates that the component is not contained. ** In the Table, "-" in the column corresponding to the (c1)/(A) value indicates that the numerical value cannot be calculated because component (A) is not contained. | | | | | | |

As shown in Table 4, also in silicone-free water-in-oil type liquid foundations containing an ultraviolet absorber and an ultraviolet scattering agent as component (B), the liquid foundation of Example 15, which contained component (A) dissolved in component (c1), showed an improvement in the SPF value and UVAPF value compared to the liquid foundation of Comparative Example 12, which did not contain component (A).

### [Industrial Applicability]

As described in detail above, the present invention can provide an emulsion composition that has a high UV protective effect and is superior in emulsification stability and feel of use.

The emulsion composition of the present invention can also be provided as a sunscreen preparation that does not use silicone-based materials or petroleum-based polymers.

This application is based on a patent application No. 2023-030390 filed in Japan, the contents of which are incorporated in full herein.

## Claims

1. A liquid emulsion composition comprising (A) an oil gelling agent having an amide group, (B) one or more selected from the group consisting of an ultraviolet absorber and an ultraviolet scattering agent, (C) one or more selected from the group consisting of divalent alcohol having 4 to 6 carbon atoms, fatty acid that is liquid at ordinary temperature, aliphatic alcohol that is liquid at ordinary temperature, an ester of straight chain saturated fatty acid and monovalent alcohol that is liquid at ordinary temperature, and an ester of aromatic carboxylic acid that is liquid at ordinary temperature, (D) an emulsifier, and (E) water.

2. The emulsion composition according to claim 1, wherein the (A) oil gelling agent having an amide group is an N-acylamino acid dialkylamide represented by the following formula (I): 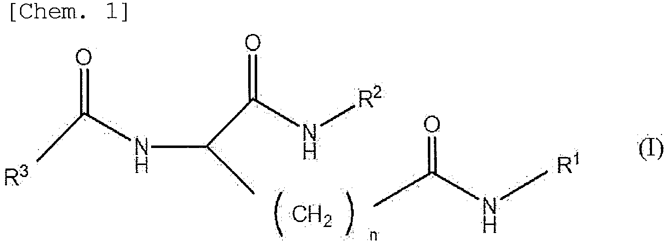 in the formula (I), R¹ and R² are the same or different and each is an alkyl group having 1 to 12 carbon atoms, R³ is a hydrocarbon group having 5 to 21 carbon atoms, and n is 1 or 2.

3. The emulsion composition according to claim 2, wherein the (A) oil gelling agent having an amide group is one or more selected from the group consisting of N-lauroyl-L-glutamic acid dibutylamide and N-2-ethylhexanoyl-L-glutamic acid dibutylamide.

4. The emulsion composition according to any one of claims 1 to 3, wherein a content of the (A) oil gelling agent having an amide group is 0.01 wt% to 3 wt%.

5. The emulsion composition according to any one of claims 1 to 3, wherein the ultraviolet absorber is an oil-soluble ultraviolet absorber.

6. The emulsion composition according to any one of claims 1 to 3, wherein the ultraviolet scattering agent is one or more selected from the group consisting of zinc oxide, titanium oxide, fine particle zinc oxide, fine particle titanium oxide, silica-treated zinc oxide, silica-treated titanium oxide, silica-treated fine particle zinc oxide, silica-treated fine particle titanium oxide, silicone-treated zinc oxide, silicone-treated titanium oxide, silicone-treated fine particle zinc oxide, silicone-treated fine particle titanium oxide, N-stearoyl-L-glutamic acid-treated zinc oxide, N-stearoyl-L-glutamic acid-treated titanium oxide, N-stearoyl-L-glutamic acid-treated fine particle zinc oxide, N-stearoyl-L-glutamic acid-treated fine particle titanium oxide, N-lauroyl-L-lysine-treated zinc oxide, N-lauroyl-L-lysine-treated titanium oxide, N-lauroyl-L-lysine-treated fine particle zinc oxide, and N-lauroyl-L-lysine-treated fine particle titanium oxide.

7. The emulsion composition according to any one of claims 1 to 3, wherein the (C) one or more selected from the group consisting of divalent alcohol having 4 to 6 carbon atoms, fatty acid that is liquid at ordinary temperature, aliphatic alcohol that is liquid at ordinary temperature, an ester of straight chain saturated fatty acid and monovalent alcohol that is liquid at ordinary temperature, and an ester of aromatic carboxylic acid that is liquid at ordinary temperature is one or more selected from the group consisting of 1,3-butanediol, 1,2-pentanediol, 1,2-hexanediol, 14-methylpentadecanoic acid, 16-methylheptadecanoic acid, 9-hexadecenoic acid, cis-9-octadecenoic acid, 2-hexyl-1-decanol, 16-methylheptadecanol, 2-octyl-1-dodecanol, 2-decyl-1-tetradecanol, cis-9-octadecen-1-ol, isopropyl myristate, isopropyl palmitate, and alkyl(C12-15) benzoate.

8. The emulsion composition according to any one of claims 1 to 3, wherein a content ratio of the component (A) and component (D) [(A):(D)] is 1:30 to 30:1 in weight ratio.

9. The emulsion composition according to any one of claims 1 to 3, further comprising (F) oily component.

10. The emulsion composition according to any one of claims 1 to 3, which is an oil-in-water type emulsion composition.

11. The emulsion composition according to any one of claims 1 to 3, which is a water-in-oil type emulsion composition.
